# EUROPEAN PATENT APPLICATION

(11) **EP 4 212 183 A1**
(43) Date of publication of application: **19.07.2023**
(21) Application number: 21866329.2
(22) Date of filing: 22.06.2021
(51) Int. Cl.: A61L 2/10, A61L 9/20

(54) **STERILIZATION SYSTEM, STERILIZER, CONTROLLER, CONTROL METHOD, AND CONTROL PROGRAM**

(30) Priority: 14.09.2020 JP 2020153590
(71) Applicant: Minebea Mitsumi Inc., Kitasaku-gun, Nagano 3890293 (JP)
(72) Inventor: IMAMURA, Shogo, Kitasaku-gun, Nagano 389-0293 (JP); SAGAWA, Yoshihiro, Kitasaku-gun, Nagano 389-0293 (JP)
(74) Representative: Zabel, Julia Elisabeth
(86) International application number: PCT/JP2021/023611
(87) International publication number: WO 2022/054368

(57) **Abstract**

A sterilization system of an embodiment includes a light source unit (21), a horizontal angle adjustment unit (4) and/or a vertical angle adjustment unit (3), and a controller (5). The light source unit (21) emits ultraviolet rays in a radiating form. The horizontal angle adjustment unit (4) adjusts a horizontal angle of the light source unit (21) by causing rotation of the light source unit (21) in a horizontal direction. The vertical angle adjustment unit (3) adjusts a vertical angle of the light source unit (21) by causing rotation of the light source unit (21) in a vertical direction. The controller (5) reads setting information including the horizontal angle and/or the vertical angle, controls the rotation caused by the horizontal angle adjustment unit (4) and/or the vertical angle adjustment unit (3), and controls irradiation of the ultraviolet rays by the light source unit (21).

## Description

### Technical Field

The present invention relates to a sterilization system, a sterilizer, a controller, a control method, and a control program.

### Background Art

A sterilizer using ultraviolet ray (ultraviolet light) irradiation to sterilize bacteria without affecting human cells has been proposed and known (see, for example, Patent Document 1 and the like).

### Citation List

### Patent Literature

Patent Document 1: JP 6025756 B

### Summary of Invention

### Technical Problem

However, the known sterilizer performs sterilization on a certain target object to be sterilized, and does not correspond to sterilization for a wide range of space such as a room and factory.

On the other hand, sterilization target objects such as viruses, including COVID-19, and bacteria float in a space and adhere to a wall forming the space and the like. For the reason, there has been no choice but to take measures, and the implementation of the measures is limited due to the necessity of changing air conditioning equipment or due to a large workload, such as replacement of air in the space or inactivation by spraying an antiseptic solution. There has been a demand for easier improvement of the environment in the space.

The present invention has been made in view of the above, and an object of the present invention is to provide a sterilization system, a sterilizer, a controller, a control method, and a control program capable of easily sterilizing a wide range of sterilization target space.

### Solution to Problem

A sterilizer according to an aspect of the present invention to solve the problem described above and achieve the object includes a light source unit, a horizontal angle adjustment unit and/or a vertical angle adjustment unit, and a controller. The light source unit emits ultraviolet rays in a radiating form. The horizontal angle adjustment unit adjusts a horizontal angle of the light source unit by causing rotation of the light source unit in a horizontal direction. The vertical angle adjustment unit adjusts a vertical angle of the light source unit by causing rotation of the light source unit in a vertical direction. The controller reads setting information including the horizontal angle and/or the vertical angle, controls the rotation caused by the horizontal angle adjustment unit and/or the vertical angle adjustment unit, and controls irradiation of the ultraviolet rays by the light source unit.

A sterilization system according to an aspect of the present invention can easily sterilize a wide range of sterilization target space.

### Brief Description of Drawings

FIG. 1 is a block diagram illustrating an example of a configuration of a sterilizer and the like according to an embodiment.
FIG. 2 is a diagram illustrating an example of a configuration in a case where a plurality of sterilizers are included.
FIG. 3 is a perspective view illustrating an example of a mechanical configuration of an example of the sterilizer.
FIG. 4 is a perspective view illustrating another example of a mechanical configuration of an example of the sterilizer.
FIG. 5 is a diagram illustrating an example of a hardware configuration of a control unit of a controller, a control unit of a terminal, or a control unit of a controller.
FIG. 6 is a diagram illustrating an example of a functional configuration of the control unit of the controller, the control unit of the terminal, or the control unit of the controller.
FIG. 7 is a diagram illustrating an example of a data structure of setting information held at the control unit of the controller, the control unit of the terminal, or the control unit of the controller.
FIG. 8 is a flowchart illustrating an example of control by the control unit of the controller, the control unit of the terminal, or the control unit of the controller.
FIG. 9 is a diagram illustrating another example of the data structure of the setting information held at the control unit of the controller, the control unit of the terminal, or the control unit of the controller.
FIG. 10 is a flowchart illustrating another example of the control by the control unit of the controller, the control unit of the terminal, or the control unit of the controller.
FIG. 11 is a diagram illustrating another example of the data structure of the setting information held at the control unit of the controller, the control unit of the terminal, or the control unit of the controller.
FIG. 12 is a diagram illustrating another example of the data structure of the setting information held at the control unit of the controller, the control unit of the terminal, or the control unit of the controller.
FIG. 13 is a diagram illustrating another example of the data structure of the setting information held at the control unit of the controller, the control unit of the terminal, or the control unit of the controller.
FIG. 14 is a diagram illustrating another example of the data structure of the setting information held at the control unit of the controller, the control unit of the terminal, or the control unit of the controller.
FIG. 15 is a diagram illustrating an example of a difference in distance from the sterilizer between positions on a floor.
FIG. 16 is a diagram illustrating another example of the data structure of the setting information held at the control unit of the controller, the control unit of the terminal, or the control unit of the controller.
FIG. 17 is a flowchart illustrating another example of the control by the control unit of the controller, the control unit of the terminal, or the control unit of the controller.
FIG. 18A is a diagram illustrating an example where a range is designated by a plurality of positions.
FIG. 18B is a diagram illustrating an example where the designated range is divided into a plurality of small ranges.
FIG. 18C is a diagram (1) illustrating a state where the sterilizer irradiates the small range obtained by the division with ultraviolet rays.
FIG. 18D is a diagram (2) illustrating a state where the sterilizer irradiates the small range obtained by the division with ultraviolet rays.
FIG. 19 is a flowchart illustrating another example of the control by the control unit of the controller, the control unit of the terminal, or the control unit of the controller.
FIG. 20A is a diagram illustrating an example where a range is designated by a plurality of positions.
FIG. 20B is a diagram illustrating a state, and in the state, positions of division points are provided between a plurality of designated positions.
FIG. 20C is a diagram (1) illustrating a state where the sterilizer irradiates each positioned with ultraviolet rays.
FIG. 20D is a diagram (2) illustrating a state where the sterilizer irradiates each positioned with ultraviolet rays.
FIG. 21 is a flowchart illustrating another example of the control by the control unit of the controller, the control unit of the terminal, or the control unit of the controller.

### Description of Embodiments

A sterilization system, a sterilizer, a controller, a control method, and a control program according to an embodiment will be described below with reference to the drawings. Note that the present invention is not limited to the embodiment described above. Furthermore, the dimensional relationships between elements, proportions of the elements, and the like in the drawings may differ from reality. The drawings may each include parts having mutually different dimensional relationships and proportions. Furthermore, the contents described in one embodiment or modified example are applied in principle to other embodiments or modification examples.

### Device Configuration, System Configuration

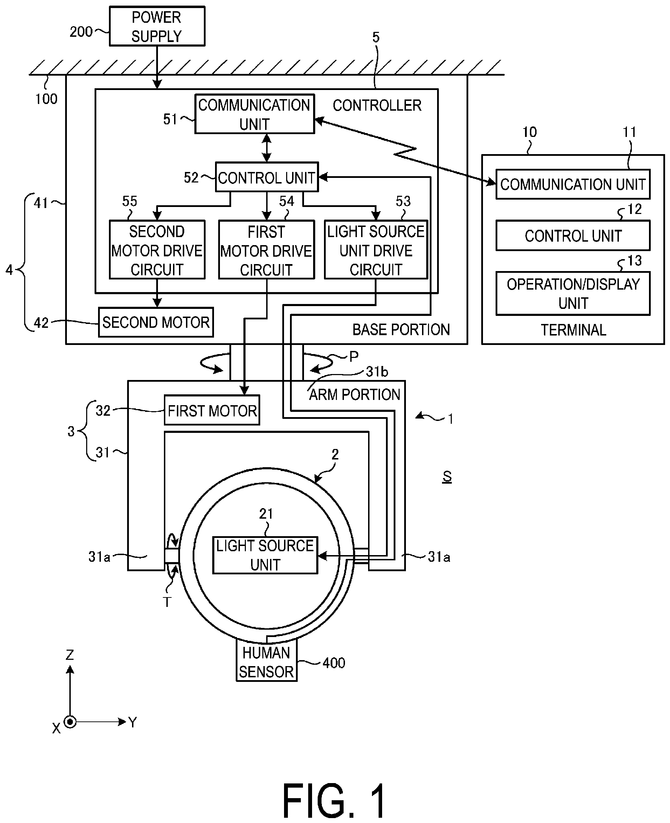

FIG. 1 is a block diagram illustrating an example of a configuration of a sterilizer 1 and the like according to an embodiment. In FIG. 1, the sterilizer 1 has a base portion 41 attached to a ceiling wall 100 forming a ceiling, for example, and includes: a main body portion 2 having a light source unit 21; a vertical angle adjustment unit 3 and a horizontal angle adjustment unit 4 rotatably supporting the main body portion 2; and a controller 5.

An X direction in FIG. 1 is a horizontal direction and is a front and back direction of the sterilizer 1A. A Y direction is a horizontal direction and is a width direction of the sterilizer 1A. A Z direction is a vertical direction orthogonal to the horizontal direction and is an up and downward direction of the sterilizer 1A. In the present embodiment, the Z direction is parallel to the vertical direction of a sterilization target space S, but is not limited to the vertical direction, and the Z direction may be inclined with respect to the vertical direction or the Z direction may be parallel to a direction orthogonal to the vertical direction.

The controller 5 controls the sterilizer 1. The controller 5 controls the light source unit 21, the vertical angle adjustment unit 3, and the horizontal angle adjustment unit 4, and is contained in an internal space of the base portion 41. The controller 5 includes a communication unit 51, a control unit 52, a light source unit drive circuit 53, a first motor drive circuit 54, and a second motor drive circuit 55. Based on electric power supplied from a power supply 200, the controller 5 performs turning ON/OFF of emission of ultraviolet rays by the light source unit 21, adjustment of the irradiation intensity, adjustment of a vertical angle of the light source unit 21 by the vertical angle adjustment unit 3, adjustment of a horizontal angle of the light source unit 21 by the horizontal angle adjustment unit 4, and the like. The hardware configuration of the controller 5 is the same as the hardware configuration of a general controller, and will be described in detail below. The power supply 200 is a commercial power supply, a generator, a battery, or the like.

The communication unit 51 transmits and receives information to and from the terminal 10 carried by a user, and, for example, receives operation information from the terminal 10, and transmits a status of the sterilizer 1, an irradiation status (for example, ON/OFF, irradiation intensity) of the light source unit 21, and an orientation status (for example, the vertical angle and the horizontal angle) of the light source unit 21 to the terminal 10. The communication unit 51 performs short-range wireless communications using Wi-Fi (registered trademark), Bluetooth (registered trademark), or the like for example.

The control unit 52 performs irradiation control on the light source unit 21, drive control on a first motor 32, drive control on a second motor 42, and the like. Here, the irradiation control includes ON/OFF control and irradiation intensity control on the light source unit 21.

The light source unit drive circuit 53 supplies electric power to the light source unit 21 based on an irradiation control signal for the light source unit 21 from the control unit 52. When the light source unit 21 is of a type capable of changing the irradiation intensity of ultraviolet rays, the light source unit drive circuit 53 also adjusts the irradiation intensity. In addition, when the light source unit drive circuit 53 is of a type capable of changing (switching) the wavelength of the ultraviolet rays emitted by the light source unit 21, the light source unit drive circuit 53 also changes (switches) the wavelength. The light source unit drive circuit 53 is electrically connected to the control unit 52 and the light source unit 21. The light source unit drive circuit 53 is included in the controller 5, but is not limited to being included in the controller 5, and may be included in the light source unit 21. When the light source unit 21 is a light source combined with light sources of different wavelength ranges, adjustment is performed so that the irradiation intensity of each of the light sources can be adjusted.

The first motor drive circuit 54 supplies driving electric power to the first motor 32 based on a drive control signal for the first motor 32 from the control unit 52, to rotate the light source unit 21 in the vertical direction. The first motor drive circuit 54 is electrically connected to the control unit 52 and the first motor 32. The first motor drive circuit 54 is included in the controller 5 but is not limited to being included in the controller 5, and may be included in the first motor 32.

The second motor drive circuit 55 supplies driving electric power to the second motor 42 based on a drive control signal for the second motor 42 from the control unit 52, to rotate the light source unit 21 in the horizontal direction. The second motor drive circuit 55 is electrically connected to the control unit 52 and the second motor 42. The second motor drive circuit 55 is included in the controller 5 but is not limited to being included in the controller 5, and may be included in the second motor 42.

The user operates the terminal 10 to operate the sterilizer 1A, and to recognize the status of the sterilizer 1A. The terminal 10 includes a communication unit 11, a control unit 12, and an operation/display unit 13. The terminal 10 may be a dedicated terminal for the sterilizer 1A, or may be a general-purpose terminal such as a smartphone, tablet, and laptop computer. The hardware configuration of the terminal 10 is similar to the hardware configuration of a general terminal, and will be described in detail below.

The communication unit 11 transmits and receives information to and from the controller 5. The communication unit 11 performs short-range wireless communications using Wi-Fi (registered trademark), Bluetooth (registered trademark), or the like for example. Note that transmission and reception of information is not limited to wireless, and may be wired transmission and reception.

The control unit 12 controls the terminal 10 and outputs operation information to the communication unit 11 based on at least an operation on the operation/display unit 13 by the user. The control unit 12 also controls display by the operation/display unit 13. The control unit 12 is electrically connected to the communication unit 11 and the operation/display unit 13. Note that the control unit 12 can take over part of processing of the control unit 52 connected via the communication unit 11 on the terminal side and the communication unit 51 on the sterilizer 1 side.

The operation/display unit 13 is used for a remote operation of the sterilizer 1A in addition to a general operation and display in the terminal 10. Although not illustrated, the operation/display unit 13 includes, for example, a switch corresponding to turning ON/OFF of the ultraviolet rays, a switch corresponding to the irradiation intensity, a switch corresponding to adjustment of the vertical angle of the light source unit 21, and a switch corresponding to adjustment of the horizontal angle of the light source unit 21. Note that the switches may be a mechanical switch such as an energized/de-energized switch or a resistance change switch, or may be an electrical switch such as a touch panel.

A human sensor 400 for detecting the presence of a person is provided at the vicinity of the main body portion 2. The human sensor 400 may detect a person in a wide range around the sterilizer 1 or may detect a person in a narrow range equivalent to the irradiation range of the ultraviolet rays.

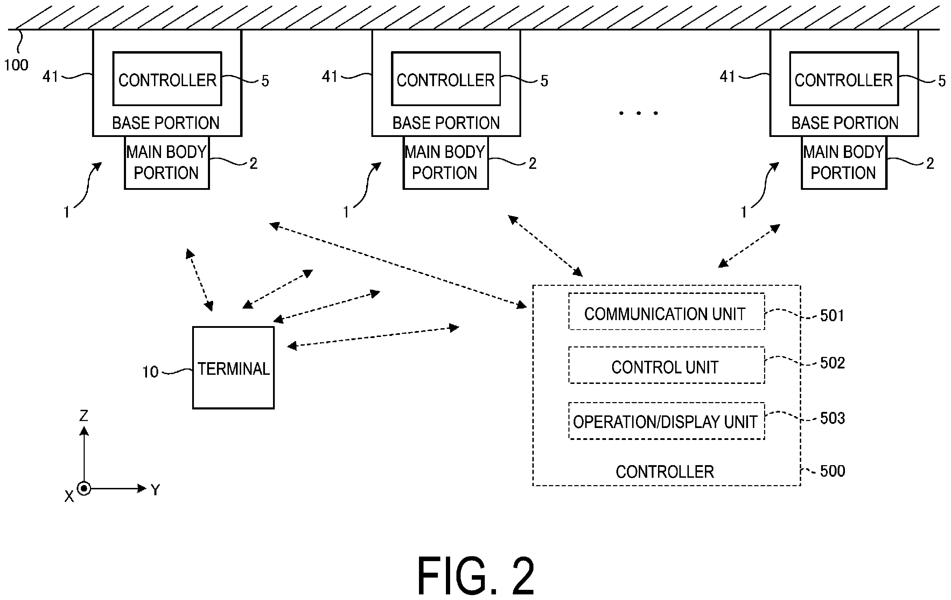

FIG. 2 is a diagram illustrating an example of a configuration in a case where a plurality of the sterilizers 1 are included. In FIG. 2, the plurality of sterilizers 1 are attached to the ceiling wall 100 forming a ceiling, for example. The controllers 5 of the respective sterilizers 1 perform control in an equal relationship, or one of the controllers 5 serves as a master to perform overall control. A controller 500 for the overall control may be provided separately from the controllers 5 of the sterilizers 1. The controller 500 includes a communication unit 501, a control unit 502, and an operation/display unit 503.

The communication unit 501 transmits and receives information to and from the controller 5 of each of the sterilizers 1. The communication unit 501 performs short-range wireless communications using Wi-Fi (registered trademark), Bluetooth (registered trademark), or the like for example. Note that transmission and reception of information is not limited to wireless, and may be wired transmission and reception.

The control unit 502 controls the controller 500 and outputs operation information to the communication unit 501 based on at least an operation on the operation/display unit 503 by the user or information set in advance. The control unit 502 is electrically connected to the communication unit 501 and the operation/display unit 503.

The operation/display unit 503 is used for a remote operation of each of the sterilizers 1 in addition to a general operation and display in the controller 500. Although not illustrated, the operation/display unit 503 includes, for example, a switch corresponding to turning ON/OFF of the ultraviolet rays, a switch corresponding to the irradiation intensity, a switch corresponding to adjustment of the vertical angle of the light source unit 21, a switch corresponding to adjustment of the horizontal angle of the light source unit 21, and a switch for setting various automatic operations. Note that the switches may be a mechanical switch such as an energized/de-energized switch or a resistance change switch, or may be an electrical switch such as a touch panel.

The terminal 10 communicates with the controller 5 of each of the sterilizers 1, with the controller 5 set to be the master, or with the controller 500, to correspond to various operations and status confirmation by the user. The terminal 10 can control each sterilizer 1 together with the controller 500 or in place of the controller 500.

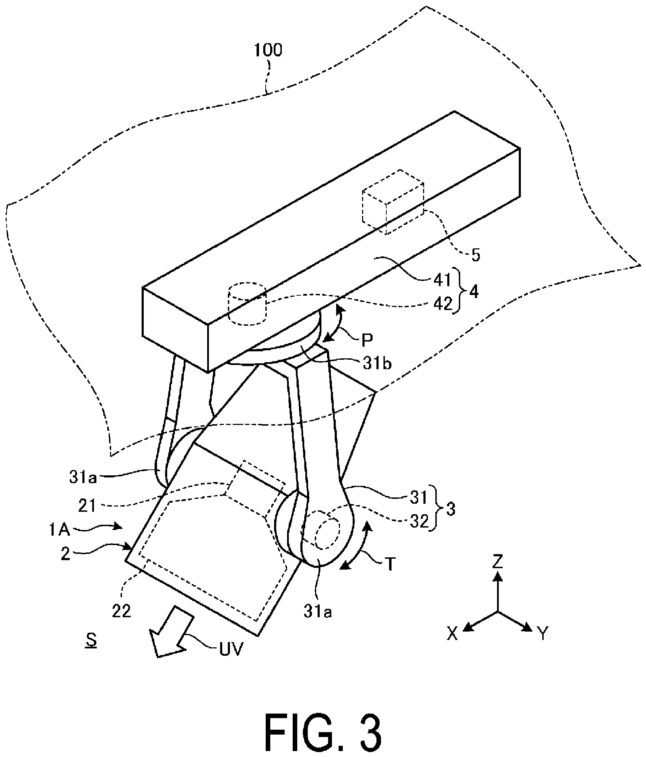

FIG. 3 is a perspective view illustrating an example of a mechanical configuration of an example of the sterilizer 1 (sterilizer 1A). As illustrated in FIG. 3, the sterilizer 1A irradiates the sterilization target space S with ultraviolet rays UV. The sterilizer 1A is attached to a wall forming a part of the sterilization target space S, that is, the ceiling wall 100 forming the ceiling in the present embodiment. Specifically, the sterilizer 1A is attached to the ceiling wall 100 via a rail or the like not illustrated, in a state of protruding from the ceiling wall 100 into the sterilization target space S. The sterilizer 1A includes the main body portion 2 with the light source unit 21 attached, the vertical angle adjustment unit 3, the horizontal angle adjustment unit 4, and the controller 5.

The light source unit 21 is attached to the main body portion 2. The main body portion 2 is formed in a substantially cylindrical shape, with an opening 22 formed at the sterilization target space S side in an axial direction and with the side opposite to the sterilization target space S closed. The main body portion 2 is supported, via a supporting shaft not illustrated, to be rotatable with respect to the vertical angle adjustment unit 3.

The light source unit 21 irradiates the sterilization target space S with the ultraviolet rays UV in a radiating form. The light source unit 21 is attached to a position opposing the opening 22 in the axial direction of the main body portion 2, in the internal space of the main body portion 2. The light source unit 21 irradiates the sterilization target space S with the ultraviolet rays UV through the opening 22. The ultraviolet rays UV emitted to the sterilization target space S by the light source unit 21 are electromagnetic waves having wavelengths different from the wavelength of visible light, and refer to electromagnetic waves having wavelengths of 190 nm to 230 nm in the present embodiment. Ultraviolet rays below 190 nm are absorbed by air, particularly oxygen in air. Thus, the amount of the ultraviolet rays reaching an object present in the sterilization target space S, for example, a human body, is greatly reduced, and the sterilization effect on the object is reduced. On the other hand, when the object present in the sterilization target space S is a human body, ultraviolet rays exceeding 230 nm may be absorbed by cells and damage DNA in the cells. The light source unit 21 of the present embodiment has, as the light source, a KrCl excimer lamp having a peak at the 222 nm for example. The ultraviolet rays UV in a radiating form may be emitted from the light source, or the ultraviolet rays of a radiating form may be obtained by a lens not illustrated from the ultraviolet rays UV in a linear form emitted from the light source.

The light source unit 21 is not limited to a light source emitting the ultraviolet rays UV in one wavelength range, and may be capable of switching between ultraviolet rays UV in different wavelength ranges. The light source unit 21 may be a combination of the light source emitting the ultraviolet rays UV, and a light source emitting electromagnetic waves, for example, visible light or infrared rays in a wavelength range different from the wavelength range of the ultraviolet rays UV. The light source unit 21 may also be a combination of a light source emitting ultraviolet rays in one wavelength range, and a light source emitting ultraviolet rays in a wavelength range different from the wavelength range. Specifically, the light source unit 21 may be a combination of a light source emitting ultraviolet rays having a wavelength of 190 nm to 230 nm, and a light source emitting ultraviolet rays having a wavelength of longer than 230 nm.

The vertical angle adjustment unit 3 rotates the light source unit 21 in the vertical direction Z, to adjust the vertical angle, that is, a tilt direction T. The vertical angle adjustment unit 3 includes an arm portion 31 and a first motor 32. The main body portion 2 is supported by the arm portion 31 to be rotatable in the vertical direction Z. The arm portion 31 is formed in a U-shape opening in the downward direction, and the main body portion 2 is supported, at both distal end portions 31a and 31a forming the opening, to be rotatable about a direction, and in the direction, the both distal end portions 31a and 31a oppose each other. The first motor 32 is a vertical actuator, and rotates the main body portion 2 with respect to the arm portion 31. The first motor 32 is attached to the arm portion 31 and rotates the main body portion 2 about the supporting shaft, that is, about the opposing direction, via a driving mechanism not illustrated. The light source unit 21 of the present embodiment is swung by the first motor 32 in a range of, for example, ±90 degrees when the downward direction is defined as 0 degrees. The vertical angle adjustment unit 3 is supported, via a supporting shaft not illustrated, to be rotatable with respect to the horizontal angle adjustment unit 4.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes the base portion 41 and the second motor 42. The main body portion 2 is supported by the base portion 41 to be rotatable in the horizontal direction, via the arm portion 31. The base portion 41 is attached to the ceiling wall 100 and formed in a cubic shape with a longitudinal direction being in the front and back direction. On the base portion 41, a distal end portion 31b of the arm portion 31 in an upper direction side is supported so that the arm portion 31 is supported to be rotatable in the vertical direction Z. The second motor 42 is a horizontal actuator and rotates the arm portion 31 with respect to the base portion 41. The second motor 42 is attached to the base portion 41, and rotates the arm portion 31 about the supporting shaft, that is, the vertical direction, via a driving mechanism not illustrated, to rotate the main body portion 2. The light source unit 21 of the present embodiment is swung by the second motor 42 in a range of, for example, ±180 degrees when any of the horizontal directions is defined as 0 degrees.

In the above description, each actuator is a motor, but the motor is not limited to the actuator, and may be a hydraulic cylinder, a pneumatic cylinder, or the like.

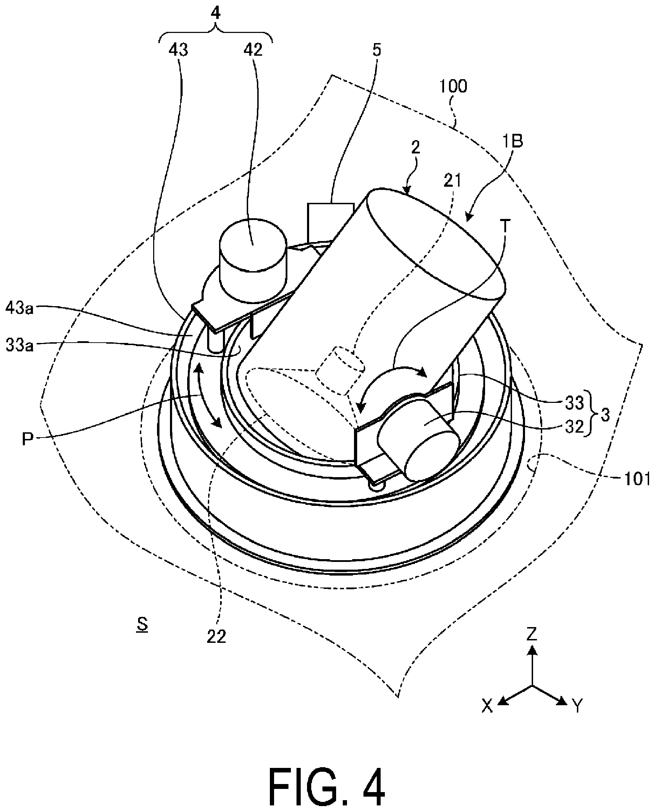

FIG. 4 is a perspective view illustrating an example of a mechanical configuration of another example of the sterilizer 1 (sterilizer 1B). In FIG. 4, the sterilizer 1B differs from the above-described sterilizer 1A in the state of attachment to the ceiling wall 100. Since the basic configuration of the sterilizer 1B is identical or substantially identical to the basic configuration of the sterilizer 1A, description of identical reference numerals will be omitted or simplified.

As illustrated in FIG. 4, the sterilizer 1B irradiates the sterilization target space S with ultraviolet rays UV. The sterilizer 1B is attached to the ceiling wall 100 forming a part of the sterilization target space S. Specifically, the sterilizer 1A is attached to the ceiling wall 100 while protruding from the ceiling wall 100 toward the side opposite to the sterilization target space S side, that is, while being buried in the ceiling wall 100. An opening 101 is formed at the ceiling wall 100, and the sterilizer 1B is attached with the sterilizer 1B, in particular, the opening 22 of the main body portion 2 exposed to the sterilization target space S through the opening 101. The sterilizer 1B includes the main body portion 2 with the light source unit 21 attached, the vertical angle adjustment unit 3, the horizontal angle adjustment unit 4, and the controller 5.

The light source unit 21 irradiating the sterilization target space S with the ultraviolet rays UV in a radiating form is attached to the main body portion 2. The main body portion 2 is formed in a substantially cylindrical shape, with an opening 22 formed at the sterilization target space S side in the axial direction and with the side opposite to the sterilization target space S side closed. The main body portion 2 is supported, via a supporting shaft not illustrated, to be rotatable with respect to the vertical angle adjustment unit 3.

The vertical angle adjustment unit 3 rotates the light source unit 21 in the vertical direction Z, to adjust the vertical angle, that is, a tilt direction T. The vertical angle adjustment unit 3 includes a sub frame portion 33 and the first motor 32. The main body portion 2 is supported by the sub frame portion 33 to be rotatable in the vertical direction Z. The sub frame portion 33, having an internal space 33a formed communicating with the outside through an opening in the upward and downward direction, is formed in a cylindrical shape with the axis in the up and down direction. In the internal space 33a of the sub frame portion 33, the main body portion 2 is supported to be rotatable about the horizontal direction. Specifically, in the sub frame portion 33, at least part of the main body portion 2 is accommodated in the internal space 33a, and the main body portion 2 is supported to be rotatable about the vertical direction. The first motor 32 is attached to the sub frame portion 33 and rotates the main body portion 2 about the supporting shaft, that is, about the opposing direction, via a driving mechanism not illustrated. The light source unit 21 of the present embodiment is swung by the first motor 32 in a range of, for example, -35 degrees to +45 degrees when the downward direction is defined as 0 degrees.

The horizontal angle adjustment unit 4 adjusts a horizontal angle, that is, adjusts a pan direction P by causing rotation of the light source unit 21 in the horizontal direction (the front and back direction X and the width direction Y). The horizontal angle adjustment unit 4 includes a base frame portion 43 and the second motor 42. The main body portion 2 is supported by the base frame portion 43 to be rotatable in the horizontal direction, via the sub frame portion 33. The base frame portion 43, with an internal space 43a formed communicating with the outside through an opening in the upward and downward direction, is attached to the ceiling wall 100 while opposing the opening 101 in the upward and downward direction, and is formed in a cylindrical shape with the axis in the upward and downward direction. In the internal space 43a of the base frame portion 43, the main body portion 2 is supported to be rotatable about the vertical direction. Specifically, in the base frame portion 43, at least part of the sub frame portion 33 is accommodated in the internal space 43a, and the sub frame portion 33 is supported to be rotatable about the vertical direction. The light source unit 21 of the present embodiment is swung by the second motor 42 in a range of, for example, +355 degrees when any of the horizontal directions is defined as 0 degrees.

In the above description, each actuator is a motor, but the motor is not limited to the actuator, and may be a hydraulic cylinder, a pneumatic cylinder, or the like.

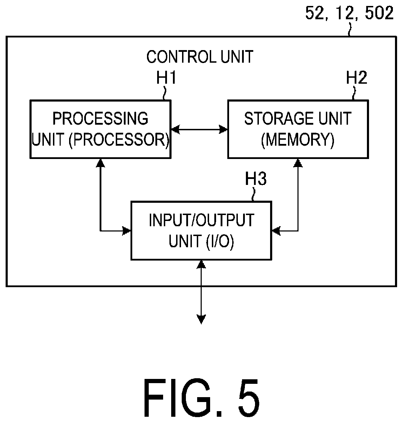

FIG. 5 is a diagram illustrating an example of a hardware configuration of the control unit 52 of the controller 5, the control unit 12 of the terminal 10, or the control unit 502 of the controller 500. In FIG. 5, the control unit 52, 12, 502 has a configuration of a general computer, and includes a processing unit (processor) H1, a storage unit (memory) H2, and an input/output unit (I/O) H3. The storage unit H2 includes a read only memory (ROM), a random access memory (RAM), a nonvolatile-RAM (NV-RAM), a solid state drive (SSD), and a hard disk drive (HDD).

The processing unit H1 executes processing based on data stored in the storage unit H2 or data input from the input/output unit H3 in accordance with a program stored in the storage unit H2. Data obtained by the processing is output from the input/output unit H3.

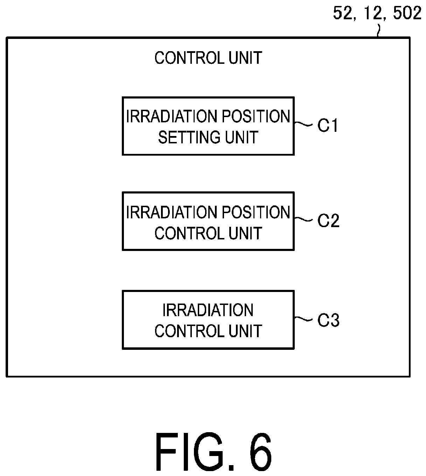

FIG. 6 is a diagram illustrating an example of a functional configuration the control unit 52 of the controller 5, the control unit 12 of the terminal 10, or the control unit 502 of the controller 500. In FIG. 6, the control unit 52, 12, 502 includes an irradiation position setting unit C1, an irradiation position control unit C2, and an irradiation control unit C3.

The irradiation position setting unit C1 has a function of setting a position or a range (a range is designated by a plurality of positions or the like) to be irradiated with ultraviolet rays for sterilization. The irradiation position control unit C2 has functions of controlling the vertical angle adjustment unit 3 and the horizontal angle adjustment unit 4 of the sterilizer 1 based on the setting information and controlling the position irradiated with ultraviolet rays. The irradiation control unit C3 has a function of turning ON and OFF the ultraviolet rays, and of changing the intensity of the ultraviolet rays (the intensity of the ultraviolet rays may or may not be changeable depending on the light sources), or changing the wavelengths of the ultraviolet rays (switching can be performed between a wavelength settable for human irradiation and a wavelength not settable for human irradiation but has high sterilization effect) based on the setting information and the like.

### Real Time Operation by User

In FIGS. 1 to 6, the user operates the terminals 10 and the like to change the status of the sterilizer 1 (1A, 1B), the irradiation status of the light source unit 21, and the orientation status of the light source unit 21. For example, when the user operates the terminal 10 to turn ON the light source unit 21, the controller 5 turns ON the light source unit 21 via the light source unit drive circuit 53, based on the irradiation control signal corresponding to the operation information corresponding to the turn ON. With the light source unit 21 turned ON, the sterilizer 1 irradiates the sterilization target space S with the ultraviolet rays UV in a radiating form, to sterilize the air in the sterilization target space S and an object in the sterilization target space S with the ultraviolet rays UV. When the user operates the terminal 10 to change the irradiation range of the ultraviolet rays UV in the sterilization target space S in the vertical direction, the controller 5 drives the first motor 32 via the first motor drive circuit 54 based on the drive control signal for the first motor 32 corresponding to the operation information corresponding to the adjustment of the vertical angle to rotate the light source unit 21 in the vertical direction. When the user operates the terminal 10 to change the irradiation range of the ultraviolet rays UV in the sterilization target space S in the horizontal direction, the controller 5 drives the second motor 42 via the second motor drive circuit 55 based on the drive control signal for the second motor 42 corresponding to the operation information corresponding to the adjustment of the horizontal angle to rotate the light source unit 21 in the horizontal direction. Thus, the user who wants to sterilize an object in the sterilization target space S rotates the light source unit 21 in the vertical direction and the horizontal direction, to move the irradiation range of the ultraviolet rays UV from the light source unit 21 to the object, and performs the sterilization.

As described above, in the sterilizer 1 (1A, 1B) according to the present embodiment, the light source unit 21 can be rotated in the horizontal direction and the vertical direction, so that the irradiation range of the ultraviolet rays UV for the sterilization target space S can be large compared with a case where the light source unit 21 cannot rotate. Thus, a wide range of the sterilization target space S can be sterilized, whereby the environment in the space can be easily improved.

The light source unit 21 emits the ultraviolet rays UV being electromagnetic waves having wavelengths of 190 nm to 230 nm. Thus, even if a person is present in the sterilization target space S and the person is irradiated with the ultraviolet rays UV from the light source unit 21, the impact of the emitted ultraviolet rays on the human body is extremely small. Therefore, even if there is a person in the sterilization target space S, the sterilization by the sterilizer 1 can be safely performed.

In the above description, the sterilizer 1 (1A, 1B) is operated by a manual operation by the user, but is not limited to being operated by the manual operation, and may be operated by automatic operation. For example, a sensor reacting to the movement of an object, such as a human sensor, for example, and having a following mode may be used. Under the following mode, the irradiation range of the ultraviolet rays UV changes to follow the movement of the object. The ultraviolet rays UV may be emitted in the irradiation direction while following a direction of location of a predetermined object, for example, a person. The control unit 52 may have a sterilization mode for an operation set in advance, and in the mode, a floor or a wall forming the sterilization target space S is entirely irradiated with the ultraviolet rays UV for example. The control unit 52 may irradiate the sterilization target space S with the ultraviolet rays UV, during predetermined time, for example, a time zone with absence of any person in the sterilization target space S.

### Automatic Irradiation based on Set Position (Fixed Irradiation Time)

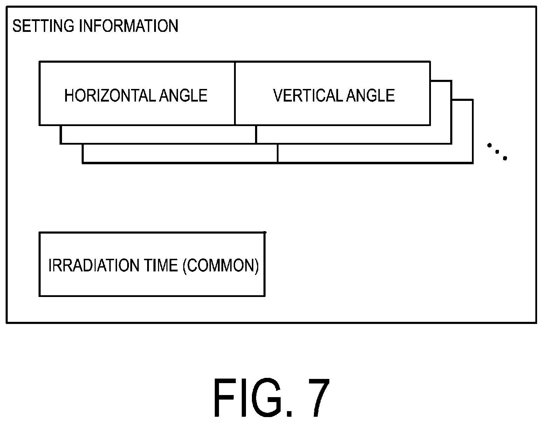

FIG. 7 is a diagram illustrating an example of a data structure of setting information held in the control unit 52 of the controller 5, the control unit 12 of the terminal 10, or the control unit 502 of the controller 500. In FIG. 7, the setting information includes a plurality of sets of a horizontal angle and a vertical angle. A set of a horizontal angle and a vertical angle indicates an irradiation direction corresponding to one position. The horizontal angle is an angle (pan) to be controlled by the horizontal angle adjustment unit 4 (FIG. 1). The vertical angle is an angle (tilt) to be controlled by the vertical angle adjustment unit 3 (FIG. 1). The setting information includes an irradiation time common to the positions.

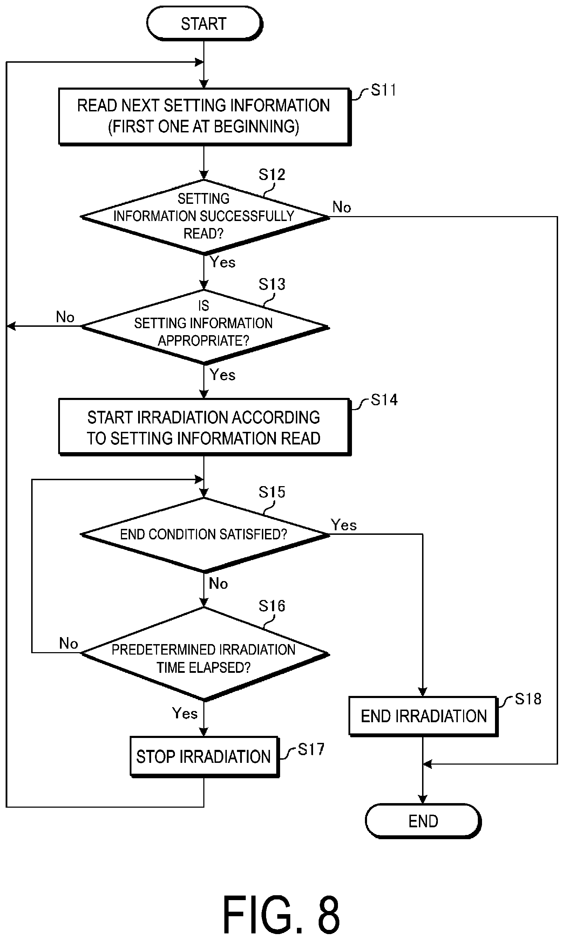

FIG. 8 is a flowchart illustrating an example of control by the control unit 52 of the controller 5, the control unit 12 of the terminal 10, or the control unit 502 of the controller 500. In FIG. 8, when the processing starts, the control unit 52 or the like reads the next setting information (first setting information at the beginning) (step S11), and determines whether the setting information was found and successfully read (step S12). When the setting information was determined not to be found or determined to be failed to be read because the final setting information has already been read (No in step S12), the control unit 52 or the like ends the processing.

When the setting information was determined to be successfully read (Yes in step S12), the control unit 52 or the like determines whether the setting information is appropriate by determining whether the setting information is not contradictive (step S13). When the setting information read was determined not to be appropriate (No in step S13), the control unit 52 or the like proceeds to reading of the next setting information (step S 11).

When the setting information read was determined to be appropriate (Yes in step S13), the control unit 52 or the like starts the ultraviolet ray irradiation based on the setting information read (step S14). Specifically, the ultraviolet ray irradiation is performed with the optical axis of the light source unit 21 set to be at the horizontal angle and the vertical angle included in the setting information.

Next, the control unit 52 or the like determines whether an end condition is satisfied with an end instruction from the user or the like (step S15), and when the end condition was determined not to be satisfied (No in step S15), then determines whether common irradiation time included in the setting information has elapsed (step S16). When the common irradiation time was determined not to have elapsed yet (No in step S16), the control unit 52 or the like proceeds to the determination on the end condition (step S15).

When the common irradiation time was determined to have elapsed (Yes in step S16), the control unit 52 or the like stops the ultraviolet ray irradiation (step S17), and proceeds to the reading of the next setting information (step S11). When the end condition is determined to have been satisfied (Yes in step S15) in the determination (step S15) for the end condition, the control unit 52 or the like stops the ultraviolet ray irradiation (step S18), and ends the processing.

Through the processing described above, the sterilization can be performed with one or a plurality of positions set in advance irradiated with ultraviolet rays. Thus, a specific position such as a door knob required to be sterilized can be automatically sterilized. Furthermore, a plurality of specific positions can be continuously sterilized.

### Automatic Irradiation based on Set Position (Variable Irradiation Time)

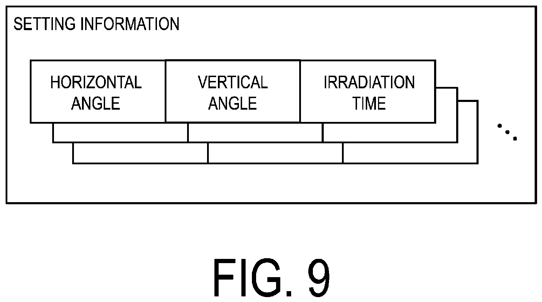

FIG. 9 is a diagram illustrating another example of the data structure of the setting information held in the control unit 52 of the controller 5, the control unit 12 of the terminal 10, or the control unit 502 of the controller 500. In FIG. 9, the setting information includes a plurality of sets of a horizontal angle, a vertical angle, and irradiation time. The horizontal angle and the vertical angle are the same as the horizontal angle and the vertical angle in FIG. 7. The irradiation time is irradiation time of ultraviolet rays for a position specified by the horizontal angle and the vertical angle. The sterilization effect by ultraviolet ray irradiation depends on the distance from the light source unit 21, the irradiation intensity of the ultraviolet rays, and the irradiation time of the ultraviolet rays. Thus, the irradiation time is settable in accordance with the position, so that a predetermined sterilization effect can be achieved.

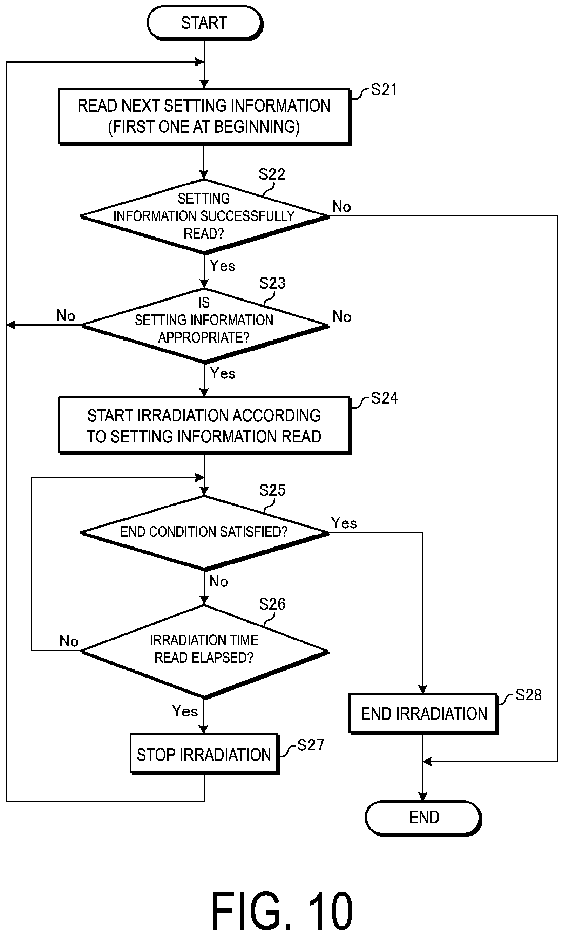

FIG. 10 is a flowchart illustrating another example of the control by the control unit 52 of the controller 5, the control unit 12 of the terminal 10, or the control unit 502 of the controller 500. In FIG. 10, when the processing starts, the control unit 52 or the like reads the next setting information (first setting information at the beginning) (step S21), and determines whether the setting information was found and successfully read (step S22). When the setting information was determined not to be found or determined to be failed to be read because the final setting information has been read (No in step S22), the control unit 52 or the like ends the processing.

When the setting information was determined to be successfully read (Yes in step S22), the control unit 52 or the like determines whether the setting information is appropriate by determining whether the setting information is not contradictive (step S23). When the setting information read is determined not to be appropriate (No in step S23), the control unit 52 or the like proceeds to reading of the next setting information (step S21).

When the setting information read is determined to be appropriate (Yes in step S23), the control unit 52 or the like starts the ultraviolet ray irradiation based on the setting information read (step S24). Specifically, the ultraviolet ray irradiation is performed with the optical axis of the light source unit 21 set to be at the horizontal angle and the vertical angle included in the setting information.

Next, the control unit 52 or the like determines whether an end condition is satisfied with an end instruction from the user or the like (step S25), and when the end condition is determined not to be satisfied (No in step S25), then determines whether irradiation time for each position included in the setting information has elapsed (step S26). When the irradiation time for each position was determined not to be elapsed yet (No in step S26), the control unit 52 or the like proceeds to the determination on the end condition (step S25).

When the irradiation time for each position was determined to have elapsed (Yes in step S26), the control unit 52 or the like stops the ultraviolet ray irradiation (step S27), and proceeds to the reading of the next setting information (step S21). When the end condition was determined to have been satisfied (Yes in step S25) in the determination (step S25) for the end condition, the control unit 52 or the like stops the ultraviolet ray irradiation (step S28), and ends the processing.

Through the processing described above, the sterilization can be performed through ultraviolet ray irradiation with the irradiation time changed for each of one or a plurality of positions set in advance. Thus, the desired sterilization effect can be achieved at a position away from the light source unit 21 or a position where careful sterilization is required. Furthermore, the desired sterilization effect can be collectively achieved for a plurality of positions. Specifically, variation of the sterilization effect depending on the distance between the light source unit 21 and the desired position can be suppressed with the irradiation time for the irradiation for a position relatively distant from the light source unit 21 set to be shorter than the irradiation time for the position close to the light source unit 21.

### Other Examples of Setting Information

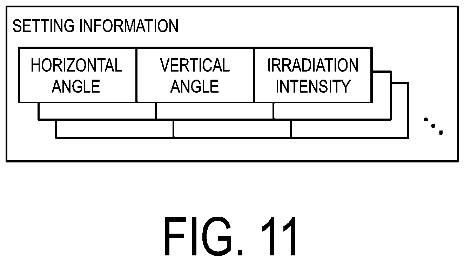

FIGS. 11 to 14 are diagrams illustrating other examples of the data structure of the setting information held in the control unit 52 of the controller 5, the control unit 12 of the terminal 10, or the control unit 502 of the controller 500.

In FIG. 11, the irradiation intensity can be set instead of the irradiation time in the setting information of FIG. 9. Although not illustrated, the setting information includes common irradiation time. The example is applicable to a case where the light source unit 21 can control the irradiation intensity of the ultraviolet rays. The processing flow is similar to the processing flow illustrated in FIG. 8.

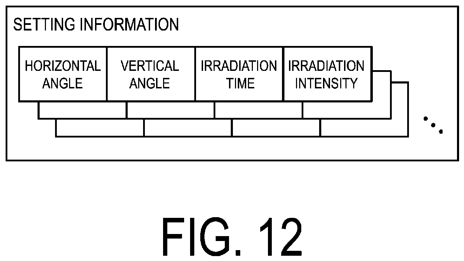

In FIG. 12, the irradiation intensity is added to the irradiation time in the setting information of FIG. 9. The processing flow is similar to the processing flow illustrated in FIG. 10.

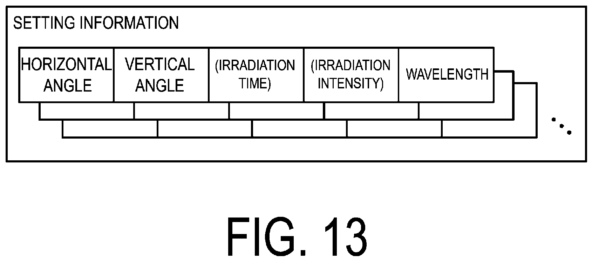

In FIG. 13, a wavelength is added to the setting information of FIG. 7, 9, 11, or 12. The setting is applicable to, for example, a case where the light source unit 21 can switch between a wavelength of 222 nm allowable for emission onto a person, and a wavelength exceeding 230 nm not allowable for emission onto a person but providing high sterilization effect. The wavelength of 222 nm is set for a position with a possibility of a person being present, and the wavelength exceeding 230 nm is set for a position with no possibility of a person being present.

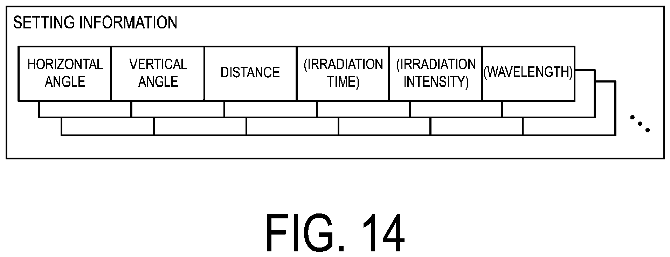

In FIG. 14, the distance from the light source unit 21 to the sterilization target is added to the setting information of FIG. 13. As described below, the distance from the sterilizer 1 to a floor surface can be calculated from the horizontal angle and the vertical angle. On the other hand, a distance to a portion on a table, wall surface, or the like cannot be easily calculated, and thus is settable. Since the irradiation time and the irradiation intensity can be calculated from the distance, the irradiation time and the irradiation intensity can be omitted.

### Example of Automatic Setting for Irradiation Time, Irradiation Intensity, and Distance

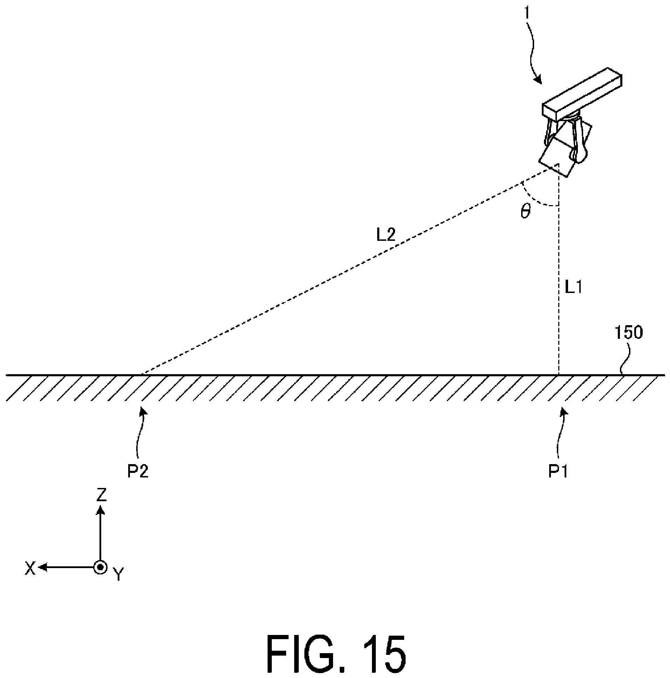

FIG. 15 is a diagram illustrating an example of a difference in distance L1, L2 from the sterilizer 1 between positions P1 and P2 on a floor 150. For example, if the distance L1 to the position P1 directly below the sterilizer 1 (being substantially the height of the ceiling) is set in advance, the distance L2 to the distant position P2 can be calculated by using a trigonometric function (L2 = L1/cosθ) based on an angle θ formed by the line segments of the distances L1 and L2. Even if the distance L1 to the position P1 directly below the sterilizer 1 is unknown, how much the irradiation time or the irradiation intensity should be increased with respect to a standard irradiation time or irradiation intensity with respect to the position P1 directly below the sterilizer 1 can be recognized based on the standard irradiation time or irradiation intensity. Thus, the irradiation time, the irradiation intensity, and the distance (only in the case of the floor surface) can be automatically set in FIGS. 9 and 11 to 14.

### Automatic Irradiation based on Set Range

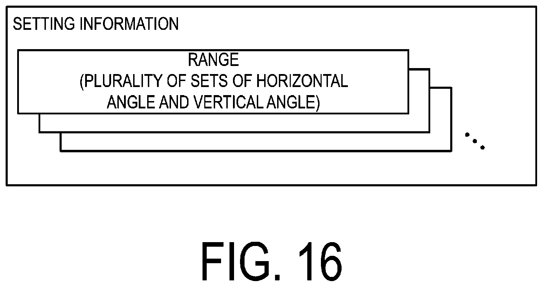

FIG. 16 is a diagram illustrating another example of the data structure of the setting information held in the control unit 52 of the controller 5, the control unit 12 of the terminal 10, or the control unit 502 of the controller 500. In FIG. 16, the setting information includes information of a plurality of ranges, and each range is specified by a plurality of sets of horizontal and vertical angles. In addition to the example where the range is specified with vertices of a polygon defining the range, other various methods may be used for specifying the range such as using the position of the center point and radius. In addition, the setting information can include irradiation time common to a plurality of pieces of setting information as illustrated in FIG. 7, as well as irradiation time, irradiation intensity, wavelength, and distance, for each setting information as illustrated in FIGS. 9 and 11 to 14.

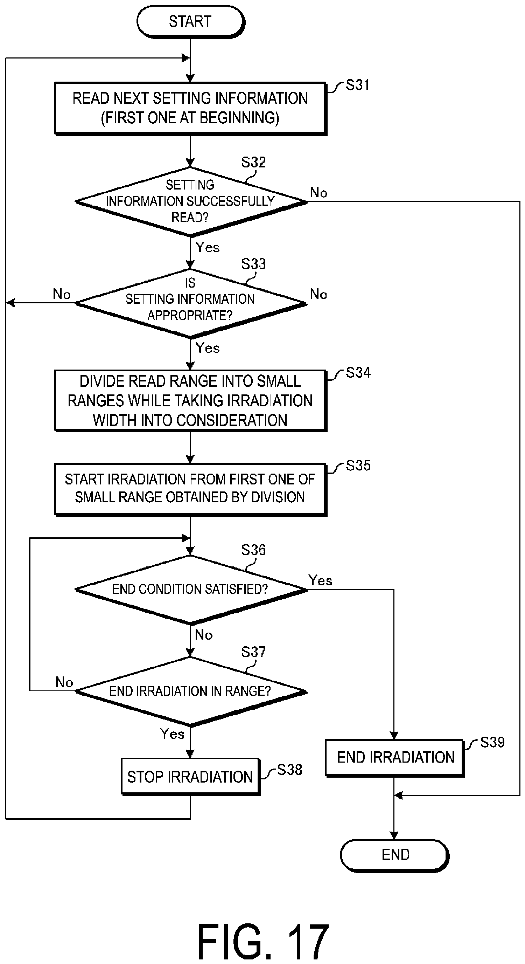

FIG. 17 is a flowchart illustrating another example of the control by the control unit 52 of the controller 5, the control unit 12 of the terminal 10, or the control unit 502 of the controller 500. In FIG. 17, when the processing starts, the control unit 52 or the like reads the next setting information (first setting information at the beginning) (step S31), and determines whether the setting information was found and successfully read (step S32). When the setting information was determined not to be found or determined to be failed to be read because the final setting information has been read (No in step S32), the control unit 52 or the like ends the processing.

When the setting information was determined to be successfully read (Yes in step S32), the control unit 52 or the like determines whether the setting information is appropriate by determining whether the setting information is not contradictive (step S33). When the setting information read was determined not to be appropriate (No in step S33), the control unit 52 or the like proceeds to reading of the next setting information (step S31).

When the setting information read was determined to be appropriate (Yes in step S33), the control unit 52 or the like divides the range in the setting information read into small ranges while taking the irradiation width of the ultraviolet rays into consideration (step S34), and starts the ultraviolet ray irradiation based on the setting information from the first one of the small ranges as a result of the division (step S35).

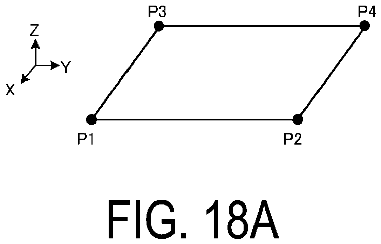

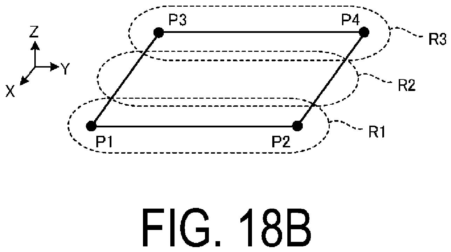

Now, an example of the division into the small ranges and ultraviolet ray irradiation will be described with reference to FIGS. 18A to 18D. FIG. 18A is a diagram illustrating an example where a range is designated by a plurality of positions P1 to P4. Here, a rectangular range is assumed to be designated by the positions P1 to P4. FIG. 18B is a diagram illustrating an example where the designated range is divided into a plurality of small ranges R1 to R3. The division is assumed to have resulted in the small range R1 extending linearly from the position P1 to the position P2, the small range R2 arranged side by side with the small range R1, and the small range R3 extending linearly from the position P3 to the position P4.

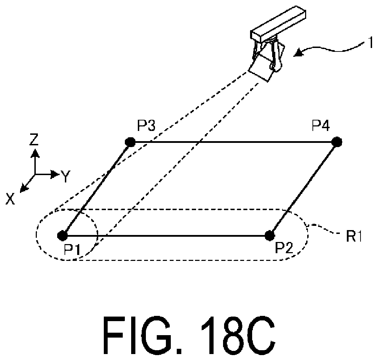

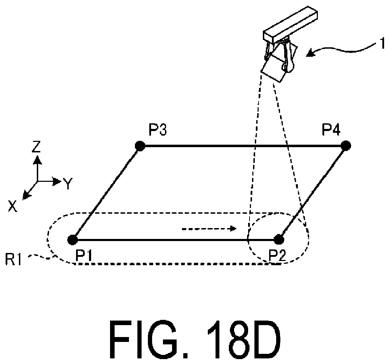

FIG. 18C is a diagram (1) illustrating a state where the sterilizer 1 irradiates the small range R1 obtained by the division with ultraviolet rays, with the ultraviolet ray irradiation starting from the position P1. FIG. 18D is a diagram (2) illustrating a state where the sterilizer 1 irradiates the small range R1 obtained by the division with ultraviolet rays, and illustrates a state where the ultraviolet ray irradiation has been performed from the position P1 to the position P2. Similarly, after a movement in a negative direction of the X axis by substantially the irradiation width from the position P2, the ultraviolet ray irradiation is performed for the small range R2 with a movement in a negative direction of the Y axis. The ultraviolet ray irradiation is similarly performed for the small range R3, and the ultraviolet ray irradiation for the range designated by the positions P1 to P4 ends. When the irradiation time, the irradiation intensity, the wavelength, the distance, and the like are set in the setting information, the ultraviolet ray irradiation is performed based on these settings.

Referring back to FIG. 17, the control unit 52 or the like determines whether an end condition is satisfied with an end instruction from the user or the like (step S36), and when the end condition was determined not to be satisfied (No in step S36), then determines whether irradiation within the range has been ended (step S37). When the irradiation within the range was determined to have not ended yet (No in step S37), the control unit 52 or the like proceeds to the determination on the end condition (step S36).

When the common irradiation within the range was determined to have ended (Yes in step S37), the control unit 52 or the like stops the ultraviolet ray irradiation (step S38), and proceeds to the reading of the next setting information (step S31). When the end condition was determined to have been satisfied (Yes in step S36) in the determination (step S36) for the end condition, the control unit 52 or the like stops the ultraviolet ray irradiation (step S39), and ends the processing.

Through the processing described above, the sterilization can be performed with one or a plurality of ranges set in advance irradiated with ultraviolet rays. Thus, a range required to be sterilized can be fully and automatically sterilized.

### Automatic Irradiation based on Set Range (simplified version)

In the processing of FIG. 17 described above, the range is divided into a plurality of small ranges. As a simpler method, division points such as middle points are provided between the positions (points) for designating the range, and the positions (points) are set as targets of ultraviolet ray irradiation, to execute the processing more easily.

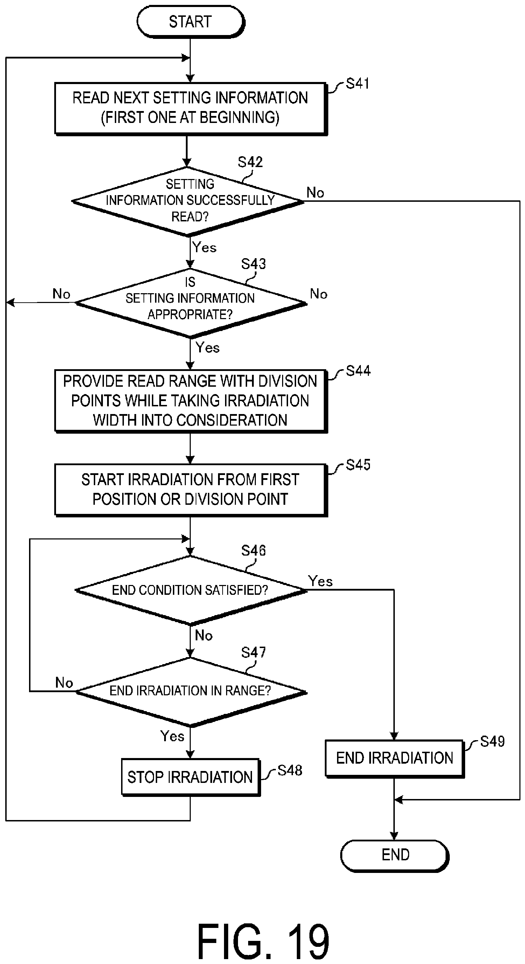

FIG. 19 is a flowchart illustrating another example of the control by the control unit 52 of the controller 5, the control unit 12 of the terminal 10, or the control unit 502 of the controller 500. Note that the setting information is similar to the setting information in FIG. 16, and can include irradiation time common to a plurality of ranges, irradiation time for each individual range, irradiation intensity, wavelength, and distance.

In FIG. 19, when the processing starts, the control unit 52 or the like reads the next setting information (first setting information at the beginning) (step S41), and determines whether the setting information was found and successfully read (step S42). When the setting information was determined not to be found or determined to be failed to be read because the final setting information has been read (No in step S42), the control unit 52 or the like ends the processing.

When the setting information was determined to be successfully read (Yes in step S42), the control unit 52 or the like determines whether the setting information is appropriate by determining whether the setting information is not contradictive (step S43). When the setting information read was determined not to be appropriate (No in step S43), the control unit 52 or the like proceeds to reading of the next setting information (step S41).

When the setting information read was determined to be appropriate (Yes in step S43), the control unit 52 or the like provides the range in the setting information read with division points while taking a rule set in advance and the irradiation width of the ultraviolet rays into consideration (step S44), and starts the ultraviolet ray irradiation based on the setting information from the first one of the positions or the division points (step S45).

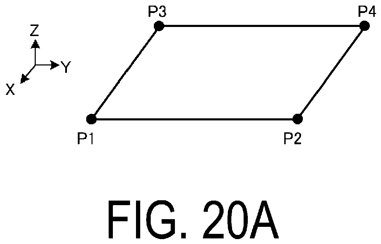

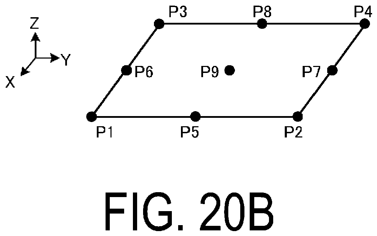

Now, an example of the provision of the division points and ultraviolet ray irradiation will be described with reference to FIGS. 20A to 20D. FIG. 20A is a diagram illustrating an example where a range is designated by a plurality of positions P1 to P4. Here, a rectangular range is assumed to be designated by the positions P1 to P4. FIG. 20B is a diagram illustrating a state of division points provided at positions P5 to P9 between a plurality of designated positions P1 to P4. Here, a division point at a position P5 is provided between the position P1 and the position P2, a division point at a position P6 is provided between the position P1 and the position P3, a division point at a position P7 is provided between the position P2 and the position P4, a division point at a position P8 is provided between the position P3 and the position P4, and a division point at a position P9 is provided between the position P5 and the position P8 (or the position P6 and the position P7).

As a rule not taking the irradiation width of the ultraviolet rays into consideration, for example, the range may be set (designated) in accordance with a parameter designated by the user, with only the designated positions being the irradiation target if the parameter is "1", with a division point provided between the designated position (midpoint) if the parameter is "2", and with trisecting division points provided between the designated positions if the parameter is "3".

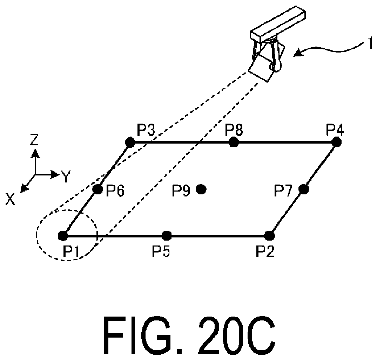

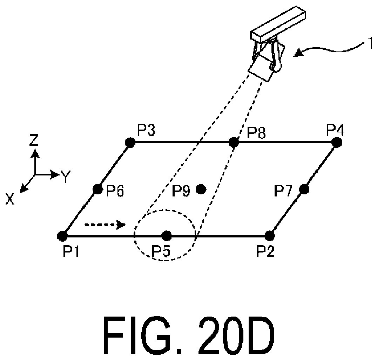

FIG. 20C is a diagram (1) illustrating a state where the sterilizer 1 irradiates each position with ultraviolet rays, with the ultraviolet ray irradiation starting from the position P1. FIG. 20D is a diagram (2) illustrating a state where the sterilizer 1 irradiates each position with ultraviolet rays, and illustrates a state where the position P5 is irradiated with the ultraviolet rays after the position P1. Similarly, the position P2, the position P7, the position P9, the position P6, the position P3, the position P8, and the position P4 are irradiated with the ultraviolet rays in the order, and the ultraviolet ray irradiation on the range designated by the positions P1 to P4 ends. When the irradiation time, the irradiation intensity, the wavelength, the distance, and the like are set in the setting information, the ultraviolet ray irradiation is performed based on these settings. The division points are also irradiated with the ultraviolet rays based on the setting information.

Referring back to FIG. 19, the control unit 52 or the like determines whether an end condition is satisfied with an end instruction from the user or the like (step S46), and when the end condition was determined not to be satisfied (No in step S46), then determines whether irradiation within the range has been ended (step S47). When the irradiation within the range was determined not to have ended yet (No in step S47), the control unit 52 or the like proceeds to the determination on the end condition (step S46).

When the common irradiation within the range was determined to have ended (Yes in step S47), the control unit 52 or the like stops the ultraviolet ray irradiation (step S48), and proceeds to the reading of the next setting information (step S41). When the end condition was determined to have been satisfied (Yes in step S46) in the determination (step S46) for the end condition, the control unit 52 or the like stops the ultraviolet ray irradiation (step S49), and ends the processing.

Through the processing described above, the sterilization can be performed with one or a plurality of ranges set in advance irradiated with ultraviolet rays. Thus, a range required to be sterilized can be fully and automatically sterilized. The predetermined range can be discretely sterilized with the division points provided, whereby the ON time of the light source unit 21 is short compared with the case of the division into small ranges, whereby the light source unit 21 can have a longer service life.

### Processing based on Time and Presence or Absence of Person

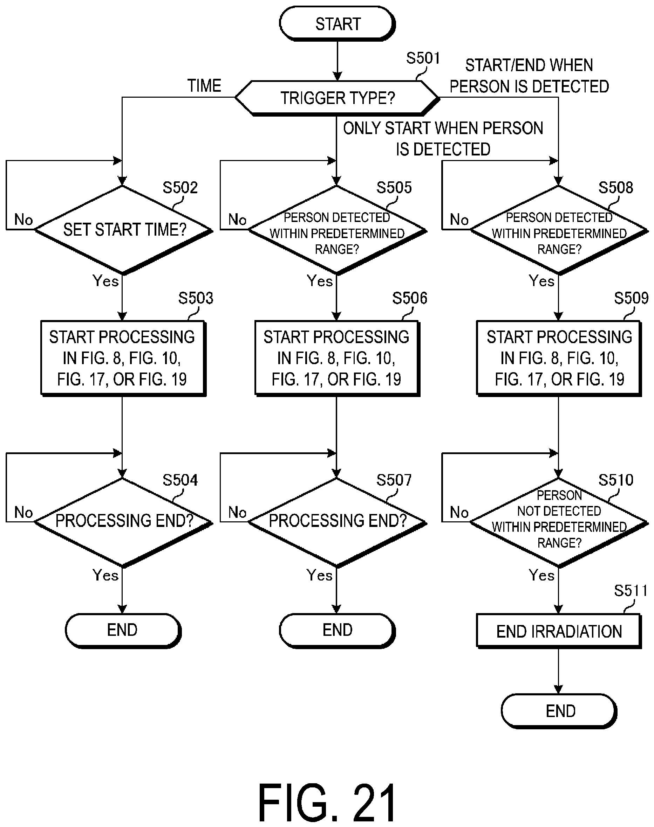

FIG. 21 is a flowchart illustrating another example of the control by the control unit 52 of the controller 5, the control unit 12 of the terminal 10, or the control unit 502 of the controller 500. In FIG. 21, upon starting the processing, the control unit 52 or the like branches the processing in accordance with the trigger type (step S501). The trigger type is set in advance by the user or the like.

When the trigger type is time, the control unit 52 or the like determines whether start time set in advance has arrived (step S502). When the start time has not arrived yet (No in step S502), the control unit 52 or the like repeats the same determination. The time may be what is known as schedule information with a day of the week or a date added. The determination on time is performed using information on a timer incorporated in the control unit 52 or the like.

When the start time set in advance was determined to have arrived (Yes in step S502), the control unit 52 or the like starts the processing of FIG. 8, 10, 17, or 19 (Step S503).

Next, the control unit 52 or the like determines whether the started processing has ended (step S504), and when the processing has not ended yet (No in step S504), repeats the same determination. When the started processing was determined to have ended (Yes in step S504), the control unit 52 or the like ends the processing.

On the other hand, when the trigger type is detection of a person for start only (start condition is detection of a person), the control unit 52 or the like determines whether a person is detected within a predetermined range (step S505), and repeats the same determination when a person is not detected yet (No in step S505). The detection of a person is performed using the human sensor 400 (FIG. 1) or the like.

When a person was determined to be detected in the predetermined range (Yes in step S505), the control unit 52 or the like starts the processing of FIG. 8, 10, 17, or 19 (step S506).

Next, the control unit 52 or the like determines whether the started processing has ended (step S507), and when the processing has not ended yet (No in step S507), repeats the same determination. When the started processing was determined to have ended (Yes in step S507), the control unit 52 or the like ends the processing.

On the other hand, when the trigger type is detection of a person for start and for end (start condition is detection of a person and end condition is the person no longer detected), the control unit 52 or the like determines whether a person is detected within a predetermined range (step S508), and repeats the same determination when a person is not detected yet (No in step S508).

When a person was determined to be detected in the predetermined range (Yes in step S508), the control unit 52 or the like starts the processing of FIG. 8, 10, 17, or 19 (step S509).

Next, the control unit 52 or the like determines whether the person is detected in the predetermined range (step S510), and when the person is still detected (No in step S510), repeats the same determination.

When the person was determined to be no longer detected in the predetermined range (Yes in step S510), the control unit 52 or the like ends the ultraviolet ray irradiation (step S511), and ends the processing.

While the case where the ultraviolet ray irradiation is performed only while the person continues to be detected has been described as one pattern, conversely, the ultraviolet ray irradiation may be performed as long as no person is detected.

With the above processing, the ultraviolet ray irradiation can be performed in accordance with desired schedule information or whether a person is present or absent.

The embodiment of the present invention has been described above, but the present invention is not limited to the embodiment described above, and various modifications are possible without departing from the spirit of the present invention.

As described above, the sterilization system according to the embodiment includes: a light source unit configured to emit ultraviolet rays in a radiating form; a horizontal angle adjustment unit configured to adjust a horizontal angle of the light source unit by causing rotation of the light source unit in a horizontal direction and/or a vertical angle adjustment unit configured to adjust a vertical angle of the light source unit by causing rotation of the light source unit in a vertical direction; and a controller configured to read setting information including the horizontal angle and/or the vertical angle, control the rotation caused by the horizontal angle adjustment unit and/or the vertical angle adjustment unit, and control irradiation of the ultraviolet rays by the light source unit. Thus, a sterilization target space can be easily sterilized over a wide range.

The setting information includes irradiation time, irradiation intensity, and/or wavelength, and the controller controls irradiation of the ultraviolet rays based on the setting information. Thus, a predetermined sterilization effect can be guaranteed.

The controller estimates a distance to a floor surface from the vertical angle in the setting information read, and changes the irradiation time, the irradiation intensity, or the wavelength. Thus, a predetermined sterilization effect can be guaranteed.

The setting information includes a range specified by a plurality of positions based on the horizontal angle and/or the vertical angle, and the controller divides the range into a plurality of small ranges, provides one or more division points between the positions, and makes the small ranges or the positions and the division points irradiated with the ultraviolet rays. Thus, a desired range can be fully sterilized.

The controller controls the irradiation of the ultraviolet rays, based on set schedule information or a result of human detection. Thus, a flexible sterilization work is possible.

Moreover, the present invention is not limited to the embodiment described above. A configuration obtained by appropriately combining the above-mentioned constituent elements is also included in the present invention. Further effects and modified examples can be easily derived by a person skilled in the art. Thus, a wide range of aspects of the present invention is not limited to the embodiment described above and may be modified variously.

### Reference Signs List

1, 1A, 1B Sterilizer, 21 Light source unit, 3 Vertical angle adjustment unit, 4 Horizontal angle adjustment unit, 5 Controller, 10 Terminal, 500 Controller, C1 Irradiation position setting unit, C2 Irradiation position control unit, C3 Irradiation control unit

## Claims

1. A sterilization system comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction; and
a controller configured to read setting information including the horizontal angle and/or the vertical angle, control the rotation caused by the horizontal angle adjustment unit and/or the vertical angle adjustment unit, and control irradiation of the ultraviolet rays by the light source unit.

2. The sterilization system according to claim 1, wherein
the setting information includes irradiation time, irradiation intensity, and/or wavelength, and
the controller controls the irradiation of the ultraviolet rays based on the setting information.

3. The sterilization system according to claim 1 or 2, wherein
the controller estimates a distance to a floor surface from the vertical angle in the setting information read, and changes irradiation time, irradiation intensity, or wavelength.

4. The sterilization system according to any one of claims 1 to 3, wherein
the setting information includes a range specified by a plurality of positions based on the horizontal angle and/or the vertical angle, and
the controller divides the range into a plurality of small ranges, provides one or more division points between the positions, and makes the small ranges or the positions and the division points irradiated with the ultraviolet rays.

5. The sterilization system according to any one of claims 1 to 4, wherein
the controller controls the irradiation of the ultraviolet rays, based on set schedule information or a result of human detection.

6. A sterilizer comprising:
a light source unit configured to emit ultraviolet rays in a radiating form;
a horizontal angle adjustment unit configured to adjust a horizontal angle of the light source unit by causing rotation of the light source unit in a horizontal direction; and/or
a vertical angle adjustment unit configured to adjust a vertical angle of the light source unit by causing rotation of the light source unit in a vertical direction; and
a controller configured to read setting information including the horizontal angle and/or the vertical angle, control the rotation caused by the horizontal angle adjustment unit and/or the vertical angle adjustment unit, and control irradiation of the ultraviolet rays by the light source unit.

7. A controller configured to perform, for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
reading of setting information including the horizontal angle and/or the vertical angle, controlling of the rotation caused by the horizontal angle adjustment unit and/or the vertical angle adjustment unit, and controlling of irradiation of the ultraviolet rays by the light source unit.

8. A control method comprising, by a computer, executing, for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
processing of reading of setting information including the horizontal angle and/or the vertical angle, controlling of the rotation caused by the horizontal angle adjustment unit and/or the vertical angle adjustment unit, and controlling of irradiation of the ultraviolet rays by the light source unit.

9. A control program causing a computer to execute, for
a light source unit configured to emit ultraviolet rays in a radiating form, and
a horizontal angle adjustment unit configured to adjust a horizontal angle of the light source unit by causing rotation of the light source unit in a horizontal direction, and/or
a vertical angle adjustment unit configured to adjust a vertical angle of the light source unit by causing rotation of the light source unit in a vertical direction,
processing of reading of setting information including the horizontal angle and/or the vertical angle, controlling of the rotation caused by the horizontal angle adjustment unit and/or the vertical angle adjustment unit, and controlling of irradiation of the ultraviolet rays by the light source unit.
